Europäisches Patentamt

European Patent Office

Office européen des brevets

(11) Publication number: **0 183 760 B1**

(12) **EUROPEAN PATENT SPECIFICATION**

(45) Date of publication of patent specification: **18.03.92**   (51) Int. Cl.⁵: **A61K 49/00**

(21) Application number: **85902602.3**

(22) Date of filing: **31.05.85**

(86) International application number:
**PCT/GB85/00234**

(87) International publication number:
**WO 85/05554 (19.12.85 85/27)**

(54) NMR CONTRAST AGENTS.

(30) Priority: **31.05.84 GB 8413849**

(43) Date of publication of application:
**11.06.86 Bulletin 86/24**

(45) Publication of the grant of the patent:
**18.03.92 Bulletin 92/12**

(84) Designated Contracting States:
**DE FR GB**

(56) References cited:
**EP-A- 0 071 564**

**Chemical Abstracts, volume 99, nr. 7, 15 August 1983, (Columbus, Ohio, US) Runge Val M. et al.: "Work in progress: potential oral and intravenous paramagnetic NMR contrast agents", see page 232, abstract number 49843k, Radiology (Easton Pa.) 1983, 147 (3), 789-91 (Eng)**

**Chemical Abstracts, volume 102, nr. 15, 15 April 1985, (Columbus, Ohio, US) Slutsky,**

**Robert A.: "Hemodynamic effects of rapid and slow infusions of manganese chloride and gadolinium-DTPA in dogs"S, see abstract number 128205n, Radiology (Easton Pa.) 1985, 154 (3), 733-5 (Eng)**

(73) Proprietor: **GUERBET S.A.**
**15, rue des Vanesses**
**F-93430 Villepinte(FR)**

(72) Inventor: **SADLER, Peter, John**
**48 Park Crescent Harrow Weald**
**Middlesex HA3 6ES(GB)**
Inventor: **HARDING, Charles, Thomas**
**Ash Trees Long Croft**
**Bovingdon Hertfordshire HP3 OJL(GB)**

(74) Representative: **Polus, Camille et al**
**c/o Cabinet Lavoix 2, Place d'Estienne d'Orves**
**F-75441 Paris Cedex 09(FR)**

**Description**

This invention relates to NMR (nuclear magnetic resonance) contrast agents, that is to say, to agents for modifying relaxation times, particularly proton relaxation times in NMR diagnosis as defined in the claims. The agents of this invention are mostly suitable for administration to humans or other animals prior to NMR imaging in vivo.

German Patent Application DE-A-3129906 describes compounds for modifying relaxation times in NMR diagnosis comprising complexes of open-chain or cyclic complex formers with paramagnetic metal ions. For human NMR diagnosis, aqueous solutions of the complexes are administered orally, neurally or intravasally, and are said to be less toxic than simple inorganic salts of the paramagnetic metals.

However, the German Patent Application leaves several problems unsolved. Toxicity of the complexes is likely to be a problem, more particularly where the complex is metabolisable. The biodistribution of the complex depends largely on the nature of the complex former, but this feature is not discussed. An NMR contrast agent is rather unlikely to be useful unless its transport and biodistribution are known and controlled with some accuracy. It is an object of this invention to overcome these problems.

The invention provides an agent for modifying relaxation times in NMR diagnosis comprising a polysaccharide having chemically linked to it an organic complexant to which is complexed a paramagnetic metal ion.

A possible advantage of the agents of this invention over simple complexes of paramagnetic metal ions is their greater influence on proton relaxation times which gives rise to better images for equivalent amounts of metal ion.

Various different polysaccharides, including chemical derivatives thereof, may be used, and will have an important influence on the properties of the agent. The polysaccharide may be water-soluble, such as dextran or dextrin, or water-insoluble, such as cellulose or Sepharose or starch. Sepharose is a bead-formed gel prepared by cross-linking agarose. Agents based on water-soluble polysaccharides may be administered orally or parenterally; agents based on water-insoluble polysaccharides are mainly suitable for oral administration.

The polysaccharide may be metabolisable or non-metabolisable by the animal to which it is administered. Compounds which are not metabolised within the time span required for NMR scanning are regarded herein as non-metabolisable, even though they may be metabolised over a much longer time span. Cellulose, Sepharose and dextran are non-metabolisable by humans, whereas starch and dextrin are metabolisable. An advantage of using a metal ion complex bound to an insoluble non-metabolisable (by humans) polysaccharide is that orally administered agent should be confined to the gastro-intestinal tract and rapidly excreted in the faeces. This is important particularly when, as is often the case, the free paramagnetic metal ion would be toxic.

Suitable paramagnetic metal ions are well known in the field and include those of the lanthanide elements with atomic numbers 58 to 70 and those of the transition metals with atomic numbers 21 to 29, 42 and 44. Preferred are Mn(II), Cu(II), Fe(II), Gd(III), Fe(III), Cr(III), Dy(III) and V(LV). Factors affecting the choice of metal ion are its paramagnetic properties, the stability of the metal ion-complexant-polysaccharide moiety, its toxicity, and the extent to which the metal ion in the complex interacts with water so as to vary the proton relaxation times.

The organic complexing agent should preferably form a complex with the paramagnetic metal ion that is stable in vivo, and this is particularly important when the free metal ion would be toxic. The complexing agent may be one which forms a chelate with the chosen metal ion. Preferred are the aminopolyacetic acids such as

Nitrilotriacetic acid

N,N,N',N'-ethylenediamine tetraacetic acid (EDTA)

N-hydroxyethyl-N,N',N'-ethylenediamine triacetic acid

N,N,N',N'',N''-diethylene triamine pentaacetic acid (DTPA)

N-hydroxyethylimino diacetic acid.

Particularly preferred are EDTA and DTPA.

Other complexing agents include those having free amino groups, such as for example aminoethyl diphosphonate (AEDP) glutamic acid and $\delta$-N-acyl-$\delta$-N-hydroxyornithine.

The complexant may be chemically linked to the polysaccharide by known chemical methods for example by the use of cyanogen bromide. The possibility arises that a complexant directly linked to polysaccharide may be sterically hindered from chelating the paramagnetic metal ion. This risk may be avoided by the use of a linker molecule between the complexant and the polysaccharide. The chemistry may be represented thus:-

2

X-OH + BrCN → X-OCN + HBr

X-OCN + $H_2N$-$(CH_2)_n$-$NH_2$ + $H_2O$

→ X-OCO-NH-$(CH_2)_n$-$NH_2$ + $NH_3$

where X is the polysaccharide residue, and n is up to 10, for example from 4 - 8.

Then:-

XOCO.NH.$(CH_2)_n$-$NH_2$ + HOCO-Y

→ XOCO.NH.$(CH_2)_n$-NHCO-Y + $H_2O$

where Y is the complexant residue.

The value of n is not critical. Indeed that the linker arm may not be needed at all in some cases. Where no linker is used, the complexant may be joined directly to the polysaccharide by known chemical techniques.

Various alternatives to cyanogen bromide are known for use to activate polysaccharides for reaction with amine-group-container linker arms (or direct with complexants). These include:-

1) 1-cyano-4-dimethylamino pyridinium tetrafluoroborate - Cyanylating agent for covalent attachment of ligands to polysaccharide resins. Febs. Letts. Vol.154 No. 1 (1983) p.209.

2) Benzoquininone.

BBA 386 (1975) 196-202.

3) Carboxymethyldextran.

J. Applied Biochem. 2, 25-35 (1980)

4) Bisepoxirane.

J. Chromatography 209, (1981) 363.

5) Divinylsulphone

Meth. Enzymology 34, (1974), 27.

6) Epichlorohydrin

J. Chromat. 51 (1970) 479. J Immunol. Meth. 58 (1983) 93-107.

7) Carbonyldiimidazole

J. Chromatography, 196, (1980) 379.

8) Periodate

Immunology 20, (1974) 1061.

9) Cyanuric chloride

Anal. Biochem. 87 (1978) 77. U.S. Patent 3,956,272 Affinity Chromatography, Elsevier Scient. Publ. Co. 1978. p.34-44 p.154, p.324-326.

10) Tosyl chloride

B. B. Res. Comm. 102 (1981) 449

Eur. J. Biochem. 112 (1980) 397

11) p-Nitro benzyl chloride.

12) 2-Aminoethyl hydrogen sulphate (dextran amine)

E. Jellum. Biochem. Pharm., 22, 1179 (1973). It is generally convenient to form the chelate with the paramagnetic ion as the final stage of the reaction sequence. This may be done simply by mixing an aqueous solution of the paramagnetic metal ion with the polysaccharide-complexant and allowing the mixture to stand, preferably with prior neutralization of some or all of the protonated metal binding sites of the complexant.

When the polysaccharide is insoluble, its physical state will depend on how it is to be administered. Finely divided material is preferably used; for example, 5 $\mu$m fibrous cellulosic material. This increases the specific surface area, and hence may increase the rate and extent of reaction with the complexant. It is this reaction that determines how much paramagnetic metal ion can be attached to unit weight of polysaccharide. Useful NMR contrast agents should contain at least one paramagnetic metal ion per 200 sugar units, perferably at least one per 100 sugar units, of the polysaccharide.

The agents may be administered orally, e.g. to visualise the gastro-intestinal tract; or parenterally e.g. to act as blood pool agents.

The following examples illustrate the invention. Examples 1 and 3 to 6 show preparation of various NMR contrast agents according to the invention. Examples 2 and 7 to 10 demonstrate properties of some of these agents in vivo and in vitro.

Example 1

Gd(III) Cellulose-linker-DTPA (GdcDTPA)

   i) Synthesis
   ii) Gd(III) binding

i) Synthesis

   (a) DTPA → DTPA ANHYDRIDE
      40g DTPA and acetic acid anhydride (38ml) in pyridine (50ml) for 24 hours at 55°C (oil bath). DTPA anhydride then purified by filtration, washed with acetic anhydride (100ml) and anhydrous ether (200ml). Dried in an oven at 50°C overnight. IR spectrum recorded
   (b) ACTIVATION OF CELLULOSE
      Cellulose (20g) and CNBr (10g in DMSO) mixed in NaOH solution pH 10.7 (100mls), pH falls rapidly, brought up to pH 10.7 with 2M NaOH addition. Mixture stirred for 25 minutes (if stirred longer then cross-linking may occur between the sugar units).
      IR spectrum of activated cellulose recorded.
   (c) ACTIVATED CELLULOSE + LINKER → CELLULOSE-LINKER
      Activated cellulose (20g) + linker (25g) [1,4-diaminobutane; 1,6-diaminohexane; 1,8-diaminooctane] in 300ml $H_2O$ and stirred for 3 hours at room temperature, maintained at pH 7 by HCl additions. Insoluble product recovered and dried.
      IR spectrum of cellulose-linker recorded.
   (d) CELLULOSE-LINKER + DTPA ANHYDRIDE → cDTPA
2 methods: 1st method (higher yield of cDTPA - see later)
      20g cellulose-linker suspended in redistilled DMF (200ml), bis anhydride DTPA (10g) added. Stirred for 16 hours at 40°C. Product collected and washed (DMF 300ml, $H_2O$ 2000ml).
2nd method
      20g cellulose-linker suspended in $H_2O$ (200ml), bis anhydride (10g) added. Stirred for 24 hours at room temperature. Product collected and washed ($H_2O$ 2000ml).

Summary

   Four cDTPA polymers have been produced:
KEY:-
   $c_4$ DTPA      cellulose-butane linker-DTPA: DMF last stage
   $c_8$ DTPA      cellulose-octane linker-DTPA: DMF last stage
   $c_6$ DTPA      cellulose-hexane linker-DTPA: DMF last stage
   $c_6$ DTPA      (aq) cellulose-hexane linker-DTPA: $H_2O$ last stage

ii) Binding of Gd(III) to $c_n$ DTPA
(where n = 4, 6 or 8)

   (1) Gd(III) binding to $c_n$DTPA
   (2) $T_1$ and $T_2$ relaxation times

(1) Gd(III) binding to $c_n$DTPA

0.5g $c_n$DTPA + $5 \times 10^{-5}$ mols Gd(III) ions (10mls of $5 \times 10^{-3}$M $Gd(NO_3)_3$ .$5H_2O$. Sample shaken for 1 hour and then spun down (4000 rpm for 3 minutes); line-width of aqueous layer recorded at 60MHz (room temperature).

4

| Tube | Sample | Line Width (Hz) | Gd(III) Bound/mol |
|---|---|---|---|
| 1 | 5mM Gd(III) | 25 | 0 |
| 2 | 0.5g $c_6$ DTPA + 5mM Gd(III) | 7 | $4.5 \times 10^{-5}$ |
| 3 | 0.5g $c_6$ DTPA (aq) + 5mM Gd(III) | 14 | $2.9 \times 10^{-5}$ |
| 4 | 0.5g $c_4$ DTPA + 5mM Gd(III) | 5 | $4.6 \times 10^{-5}$ |
| 5 | 0.5g $c_8$ DTPA + 5mM Gd(III) | 5 | $4.6 \times 10^{-5}$ |

Results imply that a higher uptake of Gd(III) occurs when DMF media are used in the final stages of $c_n$DTPA synthesis and the length of linker does not influence the number of Gd(III) ions that bind.

Gd binding was confirmed by ESR, electron spin resonance, and EM, electron microscopic x-ray analysis.

(2) Influence of $c_6$DTPA and Gdc$_6$DTPA on the $T_1$ and $T_2$ relaxation times of water

10mm NMR tube, 2ml $H_2O/D_2O$ (70:30) added. $T_1$ measured at 200MHz at room temperature using the inversion-recovery pulse sequence. 0.25g cDTPA added. Tube shaken and $T_1$ recorded. 2ml Gd(III) ions (5 $\times 10^{-3}$M Gd(NO$_3$)$_3$.5H$_2$O i.e. $10^{-5}$ mol Gd(III) ions) added. Left shaking for 1 hour, sample washed and 2 ml $H_2O/D_2O$ added. $T_1$ measured under the same previous conditions.

| Sample (2 ml) | $T_1$/s at 200 MHz |
|---|---|
| (1) $H_2O/D_2O$ | 3.74 |
| (2) $H_2O/D_2O$ + 0.25g cDTPA | 3.23 |
| (3) $H_2O/D_2O$ + 0.25g GdcDTPA | 1.30 |

0.017g Gdc$_6$DTPA evenly suspended in 0.7mls $H_2O/D_2O$(70:30) in 5 mm NMR tube. Line width recorded at 60MHz for a non-spinning sample (if spinning then a centrifugal force acts on the Gdc$_6$DTPA causing the complex to settle faster).

A line-width of 34 Hz was observed compared to 7 Hz for a similar sample of $H_2O/D_2O$ alone, showing that 0.017g of Gdc$_6$DTPA decreased the $T_2$ of water by a factor of about five.

Summary

0.5g $c_n$DTPA bind approximately 5 $\times 10^{-5}$ mol Gd(III)ions. 0.25g Gdc$_6$DTPA decreases the $T_1$ value $H_2O$ by a factor of 2.88 and decreases the $T_2$ value of $H_2O$ by a factor of ca.5. NMR studies showed that the length of linker did not influence the number of mol Gd(III) ions that bound. Calculation showed that one linker molecule attaches to every 65 sugar units of the cellulose.

Example 2

AIM

To follow $^{153}$Gd(III) uptake by $c_6$DTPA, and study [153]Gdc$_6$DTPA passage through four rat gastrointestinal tracts.

PROCEDURE

5

0.5g $c_6$DTPA soaked overnight in Tris buffer (pH 7.2). Washed via centrifugation with 3 x 10ml portions saline. 1.5ml $^{153}$GdCl$_3$/HCl in 5ml acetate buffer(pH 5.6), counted, activity determined, then added to $c_6$DTPA and left on roller for 2 hours. Solution spun down and number of counts in aqueous layer determined (therefore deduce % uptake of $^{153}$Gd(III) by $c_6$DTPA).

0.32 x 10$^{-4}$ mol Gd(NO$_3$)$_3$.5H$_2$O added to mixture, left on roller for 1 week, spun down and number of counts in aqueous medium determined. After 2 hours post innoculation of 0.5g $c_6$DTPA with 1.5ml $^{153}$GdCl$_3$ there was 91.5% incorporation of $^{153}$Gd onto $c_6$DTPA.

After 1 week post innoculation there was 63.2% incorporation of $^{153}$Gd onto DTPA.

The decrease can be attributed to dissociation of $^{153}$Gd(III) after cold Gd(III) ion addition.

After washing there was only a 0.92% loss of $^{153}$Gd, implying that the $^{153}$GdcDTPA was stable.

The $^{153}$Gdc$_6$DTPA then resuspended in 5ml H$_2$O (0.06 mCi/ml). 1ml then administered to 4 Sprague Dawley male rats (already acclimatised in metabolism cages 3 days before dosing). Each rat anaesthetised lightly with ether before oral administration (1ml of complex via a 200mm long portex 6FG catheter). After dosing count rates were taken in a twin NaI crystal counter, together with a dosing standard. Urine and faeces collected 5 hours, 24 hours and 48 hours after dosing, 2 rats dissected at 24 hours, other 2 dissected at 48 hours. The organs, urine and faeces were assayed for $^{153}$Gd in an autogammacounter.

RESULTS AND DISCUSSION

(1) By 24 hours post dosing 97% of $^{153}$Gd(III) had been excreted in the faeces, implying no degradation of $^{153}$Gdc$_6$DTPA to soluble products, which would then be excreted in the urine.

(2) After 48 hours post dosing approximately 100% of $^{153}$Gd(III) had been excreted (via faeces). Urine figures of 0.3% and 0.7% could be slight faecal contamination.

(3) The 0.4% blood figure represents a less than twice background rate.

(4) No accumulation of $^{153}$Gd(III) occurred in the organs assayed outside the GI tract at 24 or 48 hours post dosing.

| | 22 hrs sacrifice | | 46 hrs sacrifice | | mean |
|---|---|---|---|---|---|
| ANIMAL ORGAN | rat 1 | rat 2 | rat 3 | rat 4 | |
| Stomach | | 1.2% | | | |
| Small intestine | | 0.5% | | | |
| Large intestine | 0.2% | 2.8% | | | |
| Bladder/urine | | 0.3% | | 0.7% | |
| Blood | | | | 0.4% | |
| Faeces 22 hrs. | 99.8% | 95.2% | 98.2% | 94.9% | 97% |
| Faeces 48 hrs. | | | 1.8% | 3.9% | |
| Bone and other organs | 0% | 0% | 0% | 0% | |

Summary

$^{153}$Gd(III) labelling of $c_6$DTPA was approximately 60% efficient (however cold Gd(III) present in excess).

On administration into rats, via oral route, 97% of $^{153}$Gdc$_6$DTPA was excreted from the body in the faeces, implying no Gdc$_6$DTPA retention, and stability of the complex to the GI tract.

The radioactive work on $^{153}$Gdc$_6$DTPA and the data collected on Gdc$_n$DTPA relaxation properties indicates that Gdc$_n$DTPA is a potential oral contrast agent.

Example 3

Gd(III) Starch Linker DTPA (GdStDTPA)

Another water-insoluble agent was prepared by the method described in Example 1 but using starch as the starting polysaccharide in place of cellulose, and has been designated GdStDTPA.

6g starch in 300 ml $H_2O$ + 2 ml 2M NaOH; pH of solution 10.5. Stirred for 5 minutes, 2.2g CNBr (in 2 ml DMF) added dropwise, pH 10 maintained for 20 minutes by 2M NaOH addition. Then HCl added, pH lowered to 7. 3.2g 1,6-diaminohexane added, pH 7 maintained by HCl addition for 3 hours (constant stirring at room temperature) then filtered and washed with 500 ml water. The product starch-linker then taken up in 250 ml DMF (at 55°C). 2g DTPA anhydride added, mixture stirred for 12 hours at 55°C - then product starch-linker-DTPA (stDTPA) washed (300 ml DMF and 500 ml $H_2O$). Gd(III) ion binding was confirmed by electron spin resonance (ESR) analysis of a saturated GdStDTPA sample.

The $H_2O$ $T_1$ relaxation time for a Gd(III) saturated GdStDTPA derivative was shown to be 142.2 ms for a 0.12 g/ml - shorter than for GdcDTPA under the same conditions.

Example 4

Gd(III) Sepharose-linker-DTPA (GdsDTPA)

    i) Synthesis
    ii) Gd(III) binding

1) Synthesis

CNBr-activated Sepharose (1g) swollen on a sintered glass funnel for 15 minutes with 1mM HCl (10ml), then washed with 1mM HCl (200ml/g Sepharose), the HCl preserves the activity of the reactive groups which hydrolyse at high pH. The gel then washed with 5ml coupling buffer (0.25M $NaHCO_3$ + 0.5M NaCl; pH 8.5-9.0).

The gel is transferred to a solution of buffer (30ml) containing 1,6-diaminohexane (0.8g) and mixed on a rotating wheel (30 r.p.m) for 2 hours at room temperature. Use of a magnetic stirrer at this stage will break up the agarose units and a low MWT product is formed. The product is then washed on a sintered funnel; buffer (20ml) then DMF (20ml). The last wash with DMF, is critical as the next stage must be anhydrous.

The washed gel then transferred to a flask containing DMF (50ml) and DTPA anhydride (0.5g in minimal volume of DMSO). Mixture stirred for a minimum of 6 hours, on a rotating wheel at room temperature. The gel then washed (via sintered funnel) with DMF (50ml) then $H_2O$ (100ml). A solution of glutamic acid may then be administered (20ml) as this blocks any extra groups that do not have linker attached.

ii) Gd(III) binding to sDTPA

Gd(III) binding can be demonstrated, by NMR, by following the change in line-widths of the water resonance peak.

1g sDTPA placed on sintered glass funnel, 10ml Gd(III) ions added (5 x $10^{-3}$ M $Gd(NO_3)_3.5H_2O$ - known line-width). The mixture gently agitated for 1 hour. The supernatant washed through and collected, and subsequent line-width recorded. A decrease in line-width represents Gd(III) binding (i.e. the concentration of Gd(III) in the aqueous solution has decreased). $H_2O$ (5ml) added to the gel, and shaken gently for 1 hour. The supernatant washed through and the line-width recorded. The wash process repeated a second time. The line-width of 0.5g GdsDTPA/0.7mls $H_2O$ recorded, to show Gd(III) binding had occurred.

All line widths recorded at 60MHz.

Results

| Tube | Sample | Line Width (Hz) |
|------|--------|-----------------|
| 1 | $H_2O$ | 4 |
| 2 | $5 \times 10^{-3}M$ Gd(III) | 30 |
| 3 | Supernatant | 6 |
| 4 | 5ml Wash (1st) | 4 |
| 5 | 5ml wash (2nd) | 4 |

0.5g Gd sDTPA/0.7ml $H_2O$ gave a line-width of 140Hz compared to 0.5g sDTPA/0.7ml $H_2O$ which gave a line-width of 14Hz. The $T_1$ was reduced to less than 10 ms for the Gd sDTPA polymer (0.5 g/0.7 ml $H_2O$) compared to 3000 ms for sDTPA at the same concentration.

Deductions

The decrease in line-width of the $H_2O$ wash implies that 1g sDTPA binds approximately $5 \times 10^{-5}$ mol Gd(III) ions.

On washing the gel twice, no increase in the water line-width of the wash was observed, hence the complex GdsDTPA is stable.

NMR analysis of acid hydrolysate of sepharose-linker units suggests that one linker is attached to every 70 sugar units in the sepharose.

Example 5

Gd(III) Dextran-linker-(GddDTPA)

  i) Synthesis
  ii) Paramagnetic metal ion binding

i) Synthesis of dDTPA

2g dextran (18,000 MW) in a round-bottomed flask + 150ml $H_2O$ (at least 150ml, otherwise cross-linking occurs and a white ppt. forms on CNBr activation). 1g CNBr (minimal vol. DMSO) added, solution maintained at pH 10.7, by 2M NaOH addition, for 20 minutes. After 20 minutes, or when signs of cross-linking were observed (white precipitates forming), the next stage was conducted.

1,6-diaminohexane (1.2g) added to solution mixture, which then was subsequently diluted to 300mls and stirred for 3 hours (room temperature), pH maintained at pH 7.4. $H_2O$ then removed by rotary evaporation, leaving an oily product (very viscous dextran solution). DMF (100ml) added and DTPA anhydride (1g in DMSO). Mixture stirred for 12 hours at room temperature, very gently, by magnetic stirrer.

DMF then extracted by rotary evaporation. The viscous solution then poured into a presoaked dialysis sack and dialysed over 24 hours (4 x 3 litre water changes). The product then poured into a round-bottom flask and freeze-dried overnight.

ii) Binding of Paramagnetic metal ions to dDTPA 18,000 MW)

(1) Four sample tubes prepared, each containing 10ml $H_2O$ and 2.23mg $MnSO_4.4H_2O$ ($10^{-3}$M). Increasing amounts of dDTPA added to successive tubes. Line widths recorded at 60MHz. Results then tabulated. They indicate the binding of Mn(II) to dDTPA.

| Tube | dDTPA/g | Line Width (Hz) |
|------|---------|-----------------|
| 1 | 0 | 75.6 |
| 2 | 0.0054 | 65.6 |
| 3 | 0.054 | 8.4 |
| 4 | 0.09 | 7 |

NMR analysis of the hydrolysate suggests that one linker was attached to every 43 sugar residues of dextran.

8

Gadolinium binding to dDTPA polymers has been shown by ESR; having incubated a mixture of dDTPA (82,000 MW) $Gd(NO_3)_3.5H_2O$ for half an hour and then dialysing the resultant solution for 24 hours, the ESR of the dialysed solution was recorded and showed GD(III) had been retained by binding to the polymer. $H_2O$ $T_1$ measurement of the GddDTPA polymers has been recorded and compared to dextran aqueous solutions alone.

| Tube | [Gd(III)]/mM | dDTPAg/ml | T1 (200 MHz at 300° K) |
|------|--------------|-----------|------------------------|
| 1 | 0 | 0.125 | 3180 ms |
| 2 | 0.5 | 0 | 232 ms |
| 3 | 0.5 | 0.11 | 633 ms |
| 4 | 1 | 0 | 105 ms |
| 5 | 1 | 0.11 | 290 ms |
| 6 | 10 | 0 | 9 ms |
| 7 | 10 | 0.11 | 28 ms |

Results show that Gd(III) binds to dDTPA and that GddDTPA is an effective relaxation agent.

This experiment was repeated using starting dextran material of different molecular weights (9000, 18000, 82000, 110000 and 150000). This polymer type was also synthesised and characterized via carboxymethyl dextran (ref 3), tosyl chloride (ref 10), and dextran amine (ref 12).

Example 6

Gd(III) Dextan Aminoethyldiphosphonate (GddAEDP)

Dextran (82,000 MW) was activated via chloroacetic acid to carboxymethyl dextran (Ref 3). The activated dextran (3 g) was taken up in 20 mls $H_2O$. An aqueous solution of aminoethyl dihydrogen phosphate (0.5 g) was added, with adjustment of solution to pH 5-8. After 10 minutes stirring, 0.6 g of 1-ethyl-3-(3 dimethylaminopropylcarbodiimide). HCl was added, (pH 5-8 maintained). The solution was stirred for one hour, and both additions were repeated twice. After the final addition, the mixture was stirred for 12 hours and the solution then dialysed (4 x 3 litre $H_2O$ charges over 24 hours). Gd(III) Metal uptake was shown by ESR analysis on a dialysed solution mixture of $Gd(NO_3)_3. 5H_2O$ and dAEDP.

Example 7

Samples of $Gd^{153}$ dDTPA (18,000 MW) were administered orally to rabbits at dosages of 11-14 mg/kg body weight. The radioactivity was totally excreted in the faeces within 72 hours. Activity in the urine was negligible, indicating no breakdown or absorption of the complex.

Example 8

Aqueous solutions of GddDTPA (18,000 MW)(4mg/ml physiological saline) were administered to three male Sprague Dawley rats and to two rabbits at dosage levels of 10 mg/kg and 2.5 mg/kg respectively. No adverse effects were noted.

Example 9

The contrast enhancement properties of $Gdc_6$DTPA and GddDTPA in saline solution were demonstrated at 6.4 MHz by observing proton NMR images of an array of test tubes as phantoms.

Example 10

To demonstrate contrast enhancement in vivo, a total of 100 mg of GddDTPA (18,000 MW) was administered orally in ca. 50 ml of physiological saline solution to a rabbit under anaethesia. During the next few hours proton NMR images at 6.4 MHz of cross-sections showing the stomach and bowel regions were recorded using progressive saturation and inversion recovery pulse sequences. Contrast appeared in selected regions of the image due to shortening of the proton relaxation times. For example, the fluid in the stomach became white in colour whereas previously it was black and indistinguishable from the gases in the space above it (black being indicative of a low proton density or long relaxation time of protons). Similarly loops of bowel were also now distinguishable.

## Claims

1. An agent for modifying relaxation times in NMR diagnosis, comprising a polysaccharide having chemically linked to it an organic complexant to which is complexed a paramagnetic metal ion.

2. An agent as claimed in claim 1, wherein the polysaccharide is insoluble in water and not metabolisable by an animal to which the agent is to be administered.

3. An agent as claimed in claim 2, wherein the polysaccharide is cellulose, starch or Sepharose.

4. An agent as claimed in claim 1, wherein the polysaccharide is dextran.

5. An agent as claimed in anyone of claims 1 to 4, wherein the organic complexant is an aminopolyacetic acid.

6. An agent as claimed in claim 5, wherein the organic complexant is DTPA.

7. An agent as claimed in anyone of claims 1 to 6, wherein the complexant is directly linked to the polysaccharide.

8. An agent as claimed in any one of claims 1 to 6, wherein the complexant is linked to the polysaccharide by means of a linker molecule containing a chain of up to 10 carbon atoms.

9. An agent as claimed in any one of claims 1 to 8, wherein the paramagnetic metal ion is divalent Mn, trivalent Gd, or trivalent Fe.

10. An agent as claimed in any one of claims 1 to 9, wherein at least one paramagnetic metal ion is present per 100 sugar units of the polysaccharide.

11. An agent as claimed in claim 4, wherein the dextran has a molecular weight from 9,000 to 150,000.

12. An agent as claimed in claim 8, wherein the polysaccharide has been activated for the reaction with said linker by means of a chemical compound selected from cyanogen bromide, chloroacetic acid, bisepoxirane, epichlororhydrin, carbonyldiimidazole, periodate and tosyl chloride.

13. An agent as claimed in claim 7, wherein the polysaccharide has been activated for the linkage with said complexant by means of a chemical compound selected from cyanogen bromide, chloroacetic acid, bIsepoxirane, epichlorohydrin, carbonyldiimidazole, periodate, tosyl chloride and 2-aminoethyl hydrogen sulfate.

14. An agent as claimed in claim 1, wherein the paramagnetic metal ion is selectd from the lanthanide elements with atomic numbers 58 to 70 and those of the transition metals with atomic numbers 21 to 29, 42 and 44.

15. An agent according to anyone of claims 1 to 14, useable for visualizing the gastrointestinal tract.

16. An agent according to anyone of claims 1 to 14, useable as blood pool agent.

## Revendications

1. Agent de modification des temps de relaxation pour effectuer un diagnostic par RMN, comprenant un polysaccharide auguel est chimiquement lié un complexant organique avec lequel est complexé un ion de métal para-magnétique.

2. Agent selon la revendication 1, dans lequel le polysaccharide est soluble dans l'eau et n'est pas métabolisable par un animal auquel l'agent est destiné à être administré.

3. Agent selon la revendication 2, dans lequel le polysaccharide est la cellulose, l'amidon ou le Sépharose.

4. Agent selon la revendication 1, dans lequel le polysaccharide est le dextrane.

5. Agent selon l'une quelconque des revendications 1 à 4, dans lequel le complexant organique est un acide amino-polyacétique.

6. Agent selon la revendication 5, dans lequel le complexant organique est le DTPA.

7. Agent selon l'une quelconque des revendications 1 à 6, dans lequel le complexant est directement lié au polysaccharide.

8. Agent selon l'une quelconque des revendications 1 à 6, dans lequel le complexant est lié au polysaccharide, à l'aide d'une molécule de pontage contenant une chaîne de jusqu'à 10 atomes de carbone.

9. Agent selon l'une quelconque des revendications 1 à 8, dans lequel l'ion de métal para-magnétique, est le manganèse divalent, le gadolinium trivalent ou le fer trivalent.

10. Agent selon l'une quelconque des revendications 1 à 9, dans lequel au moins un ion de métal para-magnétique est présent pour 100 motifs glucidiques du polysaccharide.

11. Agent selon la revendication 4, dans lequel le dextrane a un poids moléculaire de 9000 à 150 000.

12. Agent selon la revendication 8, dans lequel le polysaccharide a été activé pour réagir avec ledit agent de pontage, à l'aide d'un composé chimique choisi parmi le bromure de cyanogène, l'acide chloroacétique, le bisépoxyrane, l'épichlorhydrine, le carbonyldiimidazole, un périodate et le chlorure de tosyle.

13. Agent selon la revendication 7, dans lequel le polysaccharide a été activé pour se lier avec le complexant, à l'aide d'un composé chimique choisi parmi le bromure de cyanogène, l'acide chloroacétique, le bisépoxyrane, l'épichlorhydrine, le carbonyldiimidazole, un périodate, le chlorure de tosyle, et l'hydrogénosulfate de 2-aminoéthyle.

14. Agent selon la revendication 1, dans lequel l'ion de métal para-magnétique est choisi parmi les éléments de lanthanide ayant des numéros atomiques allant de 58 à 70, et parmi les métaux de transition dont les numéros atomiques sont de 21 à 29, 42 et 44.

15. Agent selon l'une quelconque des revendications 1 à 14, utilisable pour visualiser le tube digestif.

16. Agent selon l'une quelconque des revendications 1 à 14, utilisable comme agent à rémanence vasculaire.

**Patentansprüche**

1. Mittel zum Ändern der Relaxationszeiten bei der NMR-Diagnose, gekennzeichnet durch ein Polysaccharid, das an einen organischen Komplexbildner chemisch gebunden ist, welcher mit einem paramagnetischen Metallion einen Komplex bildet.

2. Mittel nach Anspruch 1, dadurch gekennzeichnet, daß das Polysaccharid wasserunlöslich ist und nicht in den Stoffwechsel eines Tieres übergeht, dem das Mittel zu verabreichen ist.

3. Mittel nach Anspruch 2, dadurch gekennzeichnet, daß das Polysaccharid Zellulose, Stärke oder Sepharose ist.

4. Mittel nach Anspruch 1, dadurch gekennzeichnet, daß das Polysaccharid Dextran ist.

**5.** Mittel nach einem der Ansprüche 1 - 4, dadurch gekennzeichnet, daß der organische Komplexbildner eine Aminopolyessigsäure ist.

**6.** Mittel nach Anspruch 5, dadurch gekennzeichnet, daß der organische Komplexbildner DTPA ist.

**7.** Mittel nach einem der Ansprüche 1 - 6, dadurch gekennzeichnet, daß der Komplexbildner direkt an das Polysaccharid gebunden ist.

**8.** Mittel nach einem der Ansprüche 1 - 6, dadurch gekennzeichnet, daß der Komplexbildner mittels eines Bindungsmoleküls an das Polysaccharid gebunden ist, welches eine Kette von bis zu 10 Kohlenstoffatomen enthält.

**9.** Mittel nach einem der Ansprüche 1 - 8, dadurch gekennzeichnet, daß das paramagnetische Metallion zweiwertiges Mn, dreiwertiges Gd oder dreiwertiges Fe ist.

**10.** Mittel nach einem der Ansprüche 1 - 9, dadurch gekennzeichnet, daß mindestens ein paramagnetisches Metallion pro 100 Zuckereinheiten des Polysaccharids vorhanden ist.

**11.** Mittel nach Anspruch 4, dadurch gekennzeichnet, daß das Dextran ein Molekulargewicht von 9.000 bis 150.000 aufweist.

**12.** Mittel nach Anspruch 8, dadurch gekennzeichnet, daß das Polysaccharid mittels einer chemischen Verbindung zur Reaktion mit dem Bindungsmolekül aktiviert wurde, welche aus Bromcyan, Chloressigsäure, Bisepoxiran, Epichlorhydrin, Carbonyldiimidazol, Perjodat und Tosylchorid ausgewählt ist.

**13.** Mittel nach Anspruch 7, dadurch gekennzeichnet, daß das Polysaccharid mittels einer chemischen Verbindung zum Eingehen einer Bindung mit dem Komplexbildner aktiviert wurde, welche aus Bromcyan, Chloressigsäure, Bisepoxiran, Epichlorhydrin, Carbonyldiimidazol, Perjodat, Tosylchorid und 2-Aminoäthylhydrogensulfat ausgewählt ist.

**14.** Mittel nach Anspruch 1, dadurch gekennzeichnet, daß das paramagnetische Metallion aus Lanthanid-Elementen mit einer Ordnungszahl von 58 bis 70 und Übergangsmetallen mit einer Ordnungszahl von 21 bis 29, 42 und 44 ausgewählt ist.

**15.** Mittel nach einem der Ansprüche 1 - 14, gekennzeichnet durch seine Verwendbarkeit zum Sichtbarmachen des Magen-Darmkanals.

**16.** Mittel nach einem der Ansprüche 1 - 14, gekennzeichnet durch seine Verwendbarkeit als Blutkonzentrierungsmittel.